(19) Europäisches Patentamt
European Patent Office
Office européen des brevets

(11) Numéro de publication: 0 146 450
B1

(12) # FASCICULE DE BREVET EUROPÉEN

(45) Date de publication du fascicule du brevet: **14.01.87**

(51) Int. Cl.⁴: **C 07 C 93/06**, A 61 K 31/13

(21) Numéro de dépôt: **84402387.9**

(22) Date de dépôt: **23.11.84**

(54) **Dérivés de beta- 2-(Halogénobenzyl-)-phénoxy -éthylamine, procédé de préparation et utilisation en thérapeutique.**

(30) Priorité: **29.11.83 FR 8319056**

(43) Date de publication de la demande: **26.06.85 Bulletin 85/26**

(45) Mention de la délivrance du brevet: **14.01.87 Bulletin 87/3**

(84) Etats contractants désignés: **AT BE CH DE FR GB IT LI LU NL SE**

(56) Documents cité:
**BE-A-504 409**
**FR-A-2 372 792**

**THE JOURNAL OF ORGANIC CHEMISTRY, vol. 20, no. 8, août 1955, pages 1129-1134; NG. PH. BUU-HOI et al.: "Zinc chloride-catalyzed benzylations of phenols and naphthols"**

**Le dossier contient des informations techniques présentées postérieurement au dépôt de la demande et ne figurant pas dans le présent fascicule.**

(73) Titulaire: **INSTITUT DE RECHERCHES CHIMIQUES ET BIOLOGIQUES APPLIQUEES (I.R.C.E.B.A.) Société à responsabilité limitée dite:, 28 rue de Téhéran, F-75008 Paris (FR)**

(72) Inventeur: **Lacolle, Jean- Yves, 27 route des Suisses, F-78170 La Celle Saint Cloud (FR)**
Inventeur: **Danree, Bernard, 59 Bis boulevard Deveaux, F-78300 Poissy (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

EP 0 146 450 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne en tant que produits industriels nouveaux des dérivés de β-[2-(halogénobenzyl)-phénoxy]-éthylamine. Elle concerne également le procédé de préparation et l'utilisation en thérapeutique de ces nouveaux produits.

On sait que l'on a déjà préconisé dans le passé d'utiliser des 2-(halogénobenzyl)-4-alkylphénols (où le groupe 4-alkyle est un radical hydrocarboné ramifié, notamment un radical isopropyle, s-butyle, t-butyle ou 1,1,3,3-tétra-méthylbutyle) en tant que substances bactériostatiques, voir à cet effet US-A-3 830 852, US-A-3 855 317 et US-A-3 984 482, d'une part, et l'article de BUU-HOI et al., J. Org. Chem., 20, 1129-1134 (1955), d'autre part.

On sait en particulier que le 2-(2,4-dichlorobenzyl)-4-(1,1,3,3-tétraméthylbutyl)-phénol (qui a pour N° de code B.11 et qui est décrit à l'exemple 1 de US-A-3 830 852 précité) a été commercialisé en France en tant que médicament anti-infectieux (Dénomination Commune Internationale: "CLOFOCTOL"; nom de marque de la spécialité: "OCTOFENE"). Or, il se trouve que ce produit, qui est particulièrement efficace dans le traitement des maladies infectieuses dues aux bactéries Gram+, et ses analogues du type 2-(halogénobenzyl) 4-alkylphénol ne sont pas naturellement hydrosolubles ni hydrodispersables. La faible affinité pour l'eau constitue un inconvénient qui limite, sur le plan galénique, l'utilisation de ces produits.

On sait par ailleurs que des dérivés monohalogénés de β-[2-(halogénobenzyl)-phénoxy]-éthylamine ont déjà été décrits ou suggérés dans le passé. En particulier, l'article de BUU-HOI et al. précité décrit en tant que produits chimiques la β-[2-(4-chlorobenzyl)-4-méthylphénoxy]-N,N-diméthyl-éthylamine et son chlorhydrate, et, le brevet BE-A-504 409 englobe dans sa formule générale des sels d'addition d'acide de β-[2-(monohalogénobenzyl)-4-alkyl(inférieur)-phénoxy]-N,N-dialkyl-éthylamine mais le seul produit monohalogénobenzyle qu'il décrit, la β-[2-(4-bromobenzyl)-phénoxy]-N,N-diméthyl-éthylamine, n'est pas alkylé en position 4 du groupe phénoxy.

On sait enfin que dans FR-A-2 372 792 on a proposé en tant que substances antidépressives des ω-[2-(halogénobenzyl)-4-alkyl (en $C_1$-$C_5$)-phénoxy]-N,N-dialkyl-propyl-, butyl- ou pentylamines.

Selon l'invention, on préconise de nouveaux composés du type dichloro- et trichlorobenzyle qui sont structurellement différents de ceux de l'art antérieur précité, d'une part, et particulièrement intéressants sur le plan thérapeutique en raison notamment de leur affinité pour l'eau et de leurs propriétés anti-infectieuses et/ou antidépressives, d'autre part.

Les nouveaux composés selon l'invention, qui sont des dérivés de β-[2-(halogénobenzyl)-phénoxy]-éthylamine sont caractérisés en ce qu'ils sont choisis parmi l'ensemble constitué par

(i) les N,N dialkyl-β-[2-(polychlorobenzyl)-4-alkylphenoxy]-éthylamines répondant à la formule générale

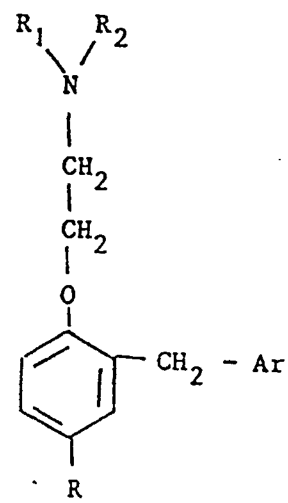

(I)

dans laquelle

R représente un groupe alkyle en $C_1$-$C_8$ à chaîne hydrocarbonée linéaire ou ramifiée,

$R_1$ et $R_2$, identiques ou différents, représentent chacun un groupe alkyle en $C_1$-$C_4$, et

Ar représente un groupe polychlorophényle de formule

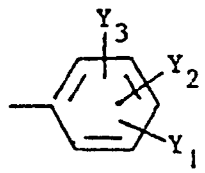

(II)

(où $Y_1$ est 4-Cl, $Y_2$ est H ou Cl et $Y_3$ est H ou Cl, un au moins des $Y_2$ et $Y_3$ etant Cl); et,

(ii) leurs sels d'addition.

Parmi les groupes alkyle R qui conviennent, on peut notamment mentionner les groupes $CH_3$, $CH_2CH_3$, $CH(CH_3)_2$, $C(CH_3)_3$, $CH(CH_3)CH_2CH_3$, $(CH_2)_3CH_3$, $CH_2C(CH_3)_3$, $CH_2CH(CH_3)CH_2CH_3$, $C(CH_3)_2CH_2C(CH_3)_3$, $C(CH_3)_2CH_2CH_3$, $(CH_2)_5CH_3$.

Par sels d'addition, on entend ici, d'une part, les sels d'addition d'acide obtenus par réaction de la base libre de formule I avec des acides minéraux ou organiques, et, d'autre part, les sels d'ammonium. Parmi les acides utilisables pour salifier la base de formule I, on peut notamment citer les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut citer les composés $XR_3$ (où X est F, Cl, Br ou I, et, $R_3$ est un groupe alkyle en $C_1$-$C_{10}$ ou un groupe benzyle).

L'invention vise donc les bases libres de formule I eer leurs sels d'addition d'acide, d'une part, et leurs sels d'ammoniun de formule

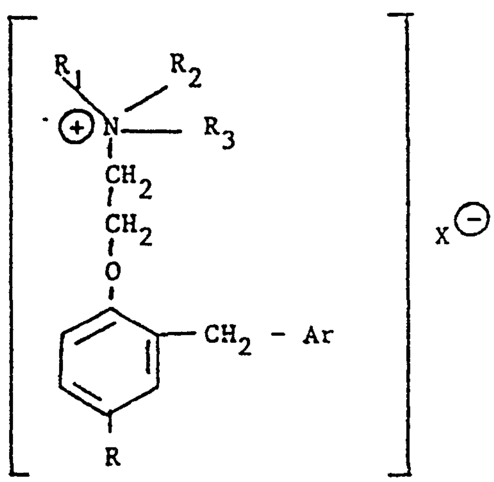

(I')

(où Ar, R, $R_1$, $R_2$, $R_3$ et X sont définis comme ci-dessus), d'autre part.

Les produits préférés selon l'invention sont ceux où $R_1 = R_2 = CH_3$ ou $CH_2CH_3$ et $Y_1$ est 4-Cl, $Y_2$ est H et $Y_3$ est 2-Cl ou 3-Cl, et qui sont représentes par la formule

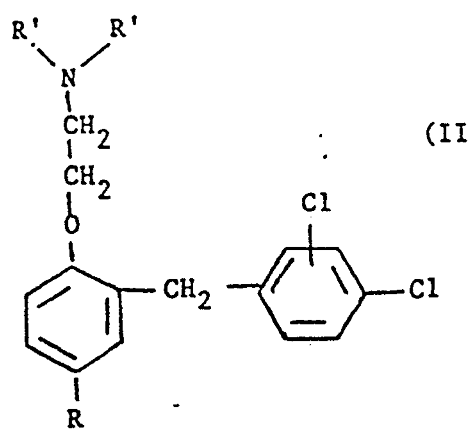

(III)

où R est un groupe alkyle en $C_1$-$C_8$, R' est $CH_3$ ou $C_2H_5$; leurs sels d'addition d'acide, et, leurs sels d'ammonium quaternaire avec $XR'_3$ (où $R'_3$ est $CH_3$, $CH_2CH_3$ ou $CH_2C_6H_5$, et, X est F, Cl, Br ou I). Les sels d'ammonium préférés sus-visés peuvent être représentés par la formule

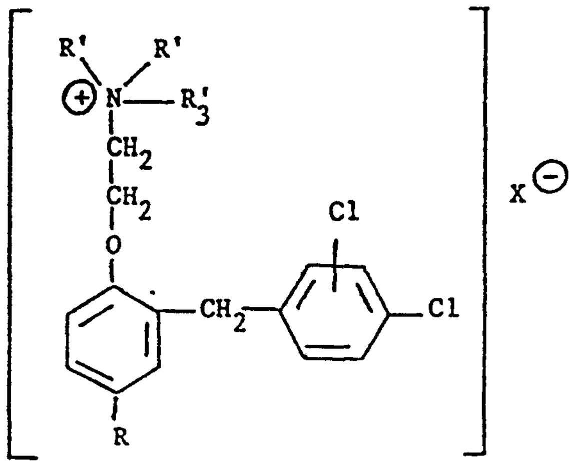

(III')

(où X, R, R' et $R_3$ sont définis comme ci-dessus. X étant avantageusement Cl, Br ou I).

Les composés selon l'invention peuvent être préparés selon une méthode connue en soi par application de mécanismes réactionnels classiques. Le procédé que l'on préconise selon l'invention consiste à faire réagir un 2-(halogénobenzyl)-4-alkylphénol de formule

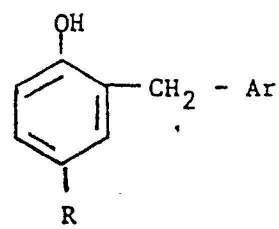

(IV)

où Ar et R sont définis comme ci-dessus, avec un halogénure de β-N,N-dialkylaminoéthyle de formule

$Hal - CH_2 - CH_2 - NR_1R_2$ (V)

(où Hal est Cl ou Br et $R_1$ et $R_2$ sont definis comme indique ci-dessus).

Cette réaction est avantageusement realisée dans un solvant inerte notamment le diméthylformamide, le tétrahydrofuranne pendant au moins 5 heures à une température inférieure ou égale à la température de reflux du milieu réactionnel à raison d'au moins 1,1 mole de V pour 1 mole de IV l'halogénure etant de préférence le chlorure (Hal = Cl).

Les bases libres de formule I, leurs sels d'addition d'acide et leurs sels d'ammonium sont utiles en thérapeutique. Ils présentent tous des propriétés antiinfectieuses en ce sens qu'ils agissent selon les doses en tant que moyens bactériostatiques, bactéricides, fongistatiques, ou, fongicides; d'une manière générale les sels d'ammonium sont plus actifs en tant que substances antiinfectieuses que les sels d'addition d'acides, ces derniers étant également plus actifs que les bases libres.

Par ailleurs, les bases libres et leurs sels d'addition d'acide présentent d'autres effets bénéfiques: ils agissent sur le système nerveux central en tant qu'agents antidépresseurs, et sont actifs vis-à-vis de l'énurésie.

Certains composés parmi les bases libres et leurs sels d'addition d'acide, tels que par exemple les produits des exemples 11 (B 795), 12 (B 805), 13 (B 809), 14 (B 785) et 15 (B 751), présentent en outre des propriétés spasmolytiques bénéfiques. Le composé le plus intéressant, en tant qu'agent spasmolytique, est le produit de l'exemple 12 (B 805).

Selon l'invention on préconise une composition thérapeutique qui est caractérisée en ce qu'elle renferme en association avec un excipient physiologiquement acceptable, au moins un composé de formule I ou l'un de ses sels d'addition, en tant que principe actif.

Bien entendu, dans une telle composition le principe actif, qui est choisi parmi les bases fibres de formule I, leurs sels d'addition non-toxiques et, le cas échéant, leurs mélanges, intervient en quantite pharmaceutiquemeut efficace.

On a consigné de façon nullement limitative dans les tableaux I, II et III qui suivent un certain nombre de composés selon l'invention; les concentrations minimales inhibitrices (CMI) de ces composés vis-à-vis d'une souche de bactérie Gram+ (Staphylococcus aureus London) d'une souche de bactérie Gram- (Eschérichia coli) et d'une souche de champignon (Candida albicans) sont données dans le tableau IV ci-après. Dans le tableau V on a consigné les CMI de déux produits selon l'invention qui sont particulièrement intéressants [composé B673

(exemple 26) et composé B 674 (exemple 29)] vis-à-vis de plusieurs souches.

Les souches utilisées sont celles des catalogues de l'"Institut Pasteur" (en abrégé "CIP") du "Centre International de Distribution de Souches et d'Information sur les Types Microbiens de Lausanne" (en abrége "La") et de l'"American Typ, Culture Collaction" (en abrégé "ATCC").

**Tableau I**

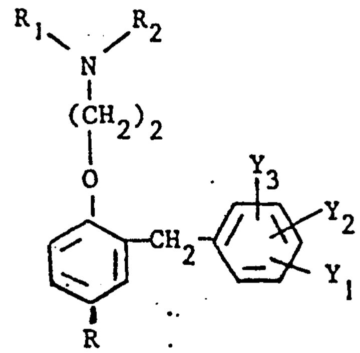

| Produit | No de Code | R | $R_1$ | $R_2$ | $Y_1$ | $Y_2$ | $Y_3$ | Eb(a) (°C) |
|---|---|---|---|---|---|---|---|---|
| Ex 1 | B 715 | CH3 | $C_2H_5$ | $C_2H_5$ | 4-Cl | H | 2-Cl | - |
| Ex 2 | B 776 | $CH_2CH_3$ | $C_2H_5$ | $C_2H_5$ | 4-Cl | H | 2-Cl | 180-190 |
| Ex 3 | B 770 | $CH(CH_3)_2$ | $C_2H_5$ | $C_2H_5$ | 4-Cl | H | 2-Cl | 200-206 |
| Ex 4 | B 803 | $CH(CH_3)CH_2CH_3$ | $C_2H_5$ | $C_2H_5$ | 4-CL | H | 2-CL | 172-174 |
| Ex 5 | B 680 | $C(CH_3)_3$ | $C_2H_5$ | $C_2H_5$ | 4-Cl | H | 2-Cl | 195-197 |
| Ex 6 | B 748 | $C(CH_3)_2CH_2CH_3$ | $C_2H_5$ | $C_2H_5$ | 4-CL | H | 2-Cl | 180-184 |
| Ex 7 | B 760 | $(CH_2)_3CH_3$ | $C_2H_5$ | $C_2H_5$ | 4-Cl | H | 2-Cl | 185-190 |
| Ex 8 | B 750 | $(CH_2)_5CH_3$ | $C_2H_5$ | $C_2H_5$ | 4-Cl | H | 2-Cl | 210-215 |
| Ex 9 | B 629 | $C(CH_3)_2CH_2C(CH_3)_3$ | $C_2H_5$ | $C_2H_5$ | 4-Cl | H | 2-Cl | - |
| Ex 10 | - | $CH(CH_3)_2$ | $CH_3$ | $CH_3$ | 4-Cl | H | 3-Cl | - |

Note (a) : Point d'ébullition sous 0,5 mmHg (soit environ 66,6 pascals)

**Tableau II**

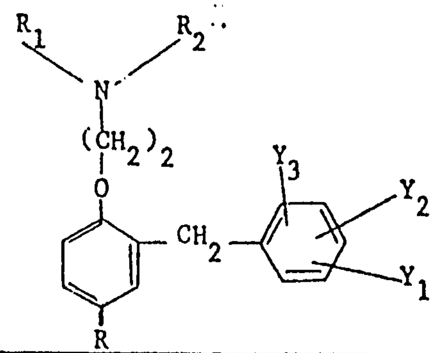

,HCl

| Produit | N° de code | R | $R_1$ | $R_2$ | $Y_1$ | $Y_2$ | $Y_3$ | F(a) (°C) |
|---|---|---|---|---|---|---|---|---|
| Ex 11 | B 795 | $CH_2CH_3$ | $C_2H_5$ | $C_2H_5$ | 4-Cl | H | 2-Cl | 155-156 |
| Ex 12 | B 805 | $CH(CH_3)_2$ | $C_2H_5$ | $C_2H_5$ | 4-Cl | H | 2-Cl | 158-159 |
| Ex 13 | B 809 | $CH(CH_3)CH_2CH_3$ | $C_2H_5$ | $C_2H_5$ | 4-Cl | H | 2-Cl | 146-147 |
| Ex 14 | B 785 | $C(CH_3)_2CH_2CH_3$ | $C_2H_5$ | $C_2H_5$ | 4-Cl | H | 2-Cl | 145-150 |
| Ex 15 | B 751 | $C(CH_3)_2CH_2C(CH_3)_3$ | $C_2H_5$ | $C_2H_5$ | 4-Cl | H | 2-Cl | 149-150 |
| Ex 16 | - | $C(CH_3)_3$ | $CH_3$ | $CH_3$ | 4-Cl | H | 2-Cl | - |
| Ex 17 | - | $CH(CH_3)_2$ | $CH_3$ | $CH_3$ | 4-Cl | H | 3-Cl | - |
| Ex 18 | - | $CH_3$ | $C_2H_5$ | $C_2H_5$ | 4-Cl | H | 2-Cl | - |
| Ex 19 | - | $C(CH_3)_3$ | $CH_3$ | $CH_3$ | 4-Cl | 3-Cl | 2-Cl | - |
| Ex 20 | - | $C(CH_3)_2CH_2CH_3$ | $CH_3$ | $CH_3$ | 4-Cl | H | 3-Cl | - |

Note . (a): point de fusion instantané.

Tableau III

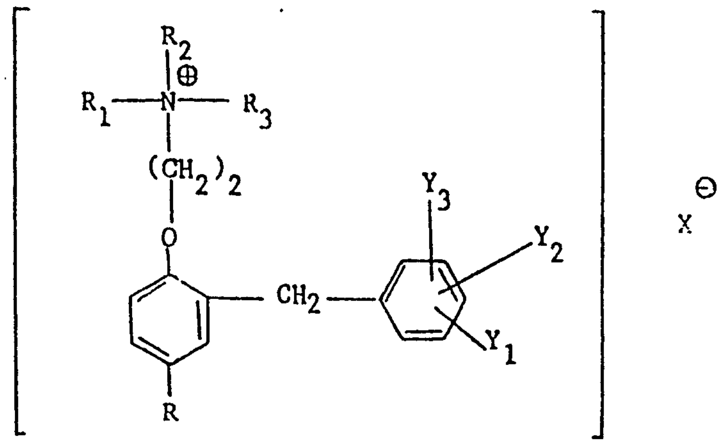

| Produit | n° de code | R | $R_1$ | $R_2$ | $R_3$ | $Y_1$ | $Y_2$ | $Y_3$ | X | F(a) (°C) |
|---|---|---|---|---|---|---|---|---|---|---|
| Ex 21 | B 794 | $C(CH_3)_2CH_2CH_3$ | $C_2H_5$ | $C_2H_5$ | $CH_3$ | 4-Cl | H | 2-Cl | I | 138-140 |
| Ex 22 | B 622 | $C(CH_3)_2CH_2C(CH_3)_3$ | $C_2H_5$ | $C_2H_5$ | $CH_3$ | 4-Cl | H | 2-Cl | I | 157-158 |
| Ex 23 | B 798 | $C(CH_3)_2CH_2CH_3$ | $C_2H_5$ | $C_2H_5$ | $(CH_2)_3CH_3$ | 4-Cl | H | 2-Cl | Br | 104-108 |
| Ex 24 | B 808 | $C(CH_3)_2CH_2C(CH_3)_3$ | $C_2H_5$ | $C_2H_5$ | $(CH_2)_3CH_3$ | 4-Cl | H | 2-Cl | Br | 167-168 |
| Ex 25 | B 852 | $C(CH_3)_3$ | $C_2H_5$ | $C_2H_5$ | $(CH_2)_3CH_3$ | 4-Cl | H | 2-Cl | Br | 124-125 |
| Ex 26 | B 673 | $CH_3$ | $C_2H_5$ | $C_2H_5$ | $CH_2C_6H_5$ | 4-Cl | H | 2-Cl | Cl | 134-136 |
| Ex 27 | B 791 | $CH_2CH_3$ | $C_2H_5$ | $C_2H_5$ | $CH_2C_6H_5$ | 4-Cl | H | 2-Cl | Cl | 125-130 |
| Ex 28 | B 774 | $CH(CH_3)_2$ | $C_2H_5$ | $C_2H_5$ | $CH_2C_6H_5$ | 4-Cl | H | 2-Cl | Cl | 116-118 |
| Ex 29 | B 674 | $C(CH_3)_3$ | $C_2H_5$ | $C_2H_5$ | $CH_2C_6H_5$ | 4-Cl | H | 2-Cl | Cl | 120-125 |

Tableau III (suite)

| Produit | n° de code | R | $R_1$ | $R_2$ | $R_3$ | $Y_1$ | $Y_2$ | $Y_3$ | X | F(a) (°C) |
|---|---|---|---|---|---|---|---|---|---|---|
| Ex 30 | B 768 | $C(CH_3)_2CH_2CH_3$ | $C_2H_5$ | $C_2H_5$ | $CH_2C_6H_5$ | 4-Cl | H | 2-Cl | Cl | 132-135 |
| Ex 31 | B 773 | $(CH_2)_3CH_3$ | $C_2H_5$ | $C_2H_5$ | $CH_2C_6H_5$ | 4-Cl | H | 2-Cl | Cl | 126-128 |
| Ex 32 | B 775 | $(CH_2)_5CH_3$ | $C_2H_5$ | $C_2H_5$ | $CH_2C_6H_5$ | 4-Cl | H | 2-Cl | Cl | 125-127 |
| Ex 33 | B 617 | $C(CH_3)_2CH_2C(CH_3)_3$ | $C_2H_5$ | $C_2H_5$ | $CH_2C_6H_5$ | 4-Cl | H | 2-Cl | Cl | 181-183 |
| Ex 34 | - | $CH_3$ | $CH_3$ | $CH_3$ | $CH_2CH_3$ | 4-Cl | H | 2-Cl | Cl | - |
| Ex 35 | - | $CH(CH_3)CH_2CH_3$ | $CH_3$ | $CH_3$ | $CH_2C_6H_5$ | 4-Cl | H | 3-Cl | Cl | - |
| Ex 36 | - | $CH(CH_3)_2$ | $CH_3$ | $CH_3$ | $CH_2C_6H_5$ | 4-Cl | H | 3-Cl | Cl | - |
| Ex 37 | - | $C(CH_3)_3$ | $CH_3$ | $CH_3$ | $CH_2C_6H_5$ | 4-Cl | H | 3-Cl | Cl | - |
| Ex 38 | - | $C(CH_3)_2CH_2C(CH_3)_3$ | $CH_3$ | $CH_3$ | $CH_2CH_3$ | 4-Cl | H | 2-Cl | F | - |
| Ex 39 | - | $CH(CH_3)CH_2CH_3$ | $CH_3$ | $CH_3$ | $CH_2CH_3$ | 4-Cl | H | 2-Cl | F | - |
| Ex 40 | - | $C(CH_3)_2CH_2CH_3$ | $C_2H_5$ | $C_2H_5$ | $CH_2CH_3$ | 4-Cl | 3-Cl | 2-Cl | F | - |

Note : (a) point de fusion instantané.

**Tableau IV**

| Produit | n° de Code | CMI (µg/ml) | | |
|---|---|---|---|---|
| | | $G^+$ | $G^-$ | C |
| Ex 1 | B 715 | 12,5 | 50 | - |
| Ex 2 | B 776 | 64 | - | 64 |
| Ex 3 | B 770 | 16 | - | 8 |
| Ex 4 | B 803 | 32 | - | 16 |
| Ex 5 | B 680 | 50 | - | - |
| Ex 6 | B 748 | 8 | - | 16 |
| Ex 7 | B 760 | 64 | 64 | 64 |
| Ex 8 | B 750 | 64 | 64 | 64 |
| Ex 9 | B 629 | 25 | - | - |
| Ex 10 | - | 16 | 64 | - |
| Ex 11 | B 795 | 8 | - | 8 |
| Ex 12 | B 805 | 4 | - | 4 |
| Ex 13 | B 809 | 8 | - | 0,5 |
| Ex 14 | B 785 | 8 | 64 | 4 |
| Ex 15 | B 751 | 16 | - | - |
| Ex 21 | B 794 | 0,125 | 8 | 0,5 |
| Ex 22 | B 622 | 0,125 | 64 | 0,25 |
| Ex 23 | B 798 | 0,06 | 4 | 0,25 |
| Ex 24 | B 808 | 0,015 | 16 | 1 |
| Ex 26 | B 673 | 0,25 | 32 | 4 |
| Ex 27 | B 791 | 0,125 | 8 | 0,5 |
| Ex 28 | B 774 | 0,015 | 8 | 0,25 |
| Ex 29 | B 674 | 0,015 | 4 | 0,125 |
| Ex 30 | B 768 | 0,004 | 4 | 0,25 |
| Ex 31 | B 773 | 0,031 | 4 | 0,125 |
| Ex 32 | B 775 | 0,125 | 16 | 0,5 |
| Ex 33 | B 617 | 0,03 | - | 1 |

<u>Notes</u> : $G^+$ = souche $Gram^+$ : <u>Staphylococcus</u> <u>aureus</u> London CIP A.238
$G^-$ = souche $Gram^-$ : <u>Escherichia</u> <u>coli</u> CIP 54.8
C = souche de champignon : <u>Candida</u> <u>albicans</u> CIP 1180

**Tableau V**

CMI (en µg/ml) vis-à-vis de plusieurs souches

| Souches | Exemple 26 (B 673) | Exemple 29 (B 674) |
|---|---|---|
| *Staphylococcus aureus* ATCC 6538 P | 0,78 | 0,78 |
| *Streptococcus pyogenes* la 147 | 0,78 | 0,78 |
| *Streptococcus pyogenes* CIP A.241 | 0,78 | 0,78 |
| *Streptococcus pyogenes* CIP 56.42 | 0,78 | 0,78 |
| *Streptococcus faecalis* CIP 53:152 | 3,125 | 0,78 |
| *Diplococcus pneumoniae* la 209 | 1,56 | 0,78 |
| *Bacillus subtilis* ATCC 6633 | 1,56 | 0,78 |
| *Corynebacterium diphteriae* CIP A.102 | 25 | 3,125 |
| *Branhamella catarrhalis* la 987 | 0,78 | 0,78 |
| *Neisseria gonorrhoeae* CIP A.52 | 0,78 | 0,78 |
| *Haemophilus influenzae* CIP 5293 | 0,78 | 0,78 |
| *Klebsiella pneumoniae* la 433 | 25 | 6,25 |
| *Streptococcus pyogenes* CIP 5641 | 0,78 | 0,78 |
| *Neisseria meningitidis* CIP 7310 | 0,78 | 0,78 |
| *Listeria monocytogenes* CIP 54153 | 1,56 | 0,78 |

D'une manière générale les composés selon l'invention sont peu toxiques et bien tolérés par l'organisme. Le tableau VI ci-après montre en particulier que les sels d'ammonium des composés de formule I sont moins toxiques que des sels d'ammonium antérieurement connus en tant que moyens anti-infectieux.

**Tableau VI**

Toxicite

| Produit | n° de code | DL-50 souris (mg/kg) |
|---|---|---|
| Ex 23 | B 798 | >1 000 |
| Ex 26 | B 673 | >1 000 |
| Ex 27 | B 791 | 850 |
| Ex 29 | B 674 | ≥1 000 |
| Ex 30 | B 768 | >1 000 |
| Ex 31 | B 773 | >1 000 |
| A-1 (a) | - | 550 |
| A-2 (b) | - | 750 |

Notes : (a) : chlorure de benzalkonium
(b) : chlorure de cétrymonium

D'autres avantages et caractéristiques de l'invention seront mieux compris à la lecture qui va suivre d'exemples de préparation nullement limitatifs, mais donnés à titre d'illustration. Préparation I

Obtention de la β-[2-(2,4-dichlorobenzyl)-4-tertiobutylphénoxyl-N ,Ndiéthyl-éthylamine.

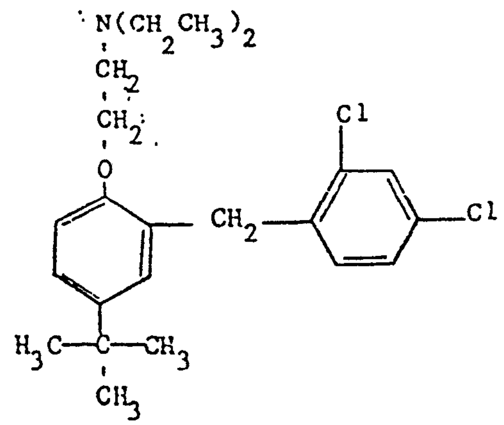

(exemple 5; n° de code; B 680);

autre nomenclature: 2- (2-diéthylamino-éthoxy)-5-tertiobutyl-2',4'-dichlorodiphénylméthane.

On fait réagir dans un réacteur un mélange constitué par 1 mole (309,18 g) de 2-(2,4-dichlorobenzyl)-4-tertiobutyl-phénol, 2,4 mole de $K_2CO_3$, 2 litres de diméthylformamide et 1,2 moles de chlorhydrate de 2-chloro-N,N-diéthyl-éthylamine, sous agitation pendant 15 heures à 120° C.

On verse le milieu réactionnel dans de l'eau saturée en NaCl extrait au moyen de $CHCl_3$, lave la phase chloroformique avec de l'eau sèche et évapore le solvant sous vide. Par distillation sous vide du résidu d'évaporation on obtient le B 680 avec un rendement de 76,1 %.

$Eb_{0,5\ mmHg}$ 195-197° C. Préparation II

Obtention du chlorure de N,N-diéthyl-N-{2-[2-(2,4-dichlorobenzyl)-4 tertiobutylphénoxy]-éthyl}-N-benzylammonium.

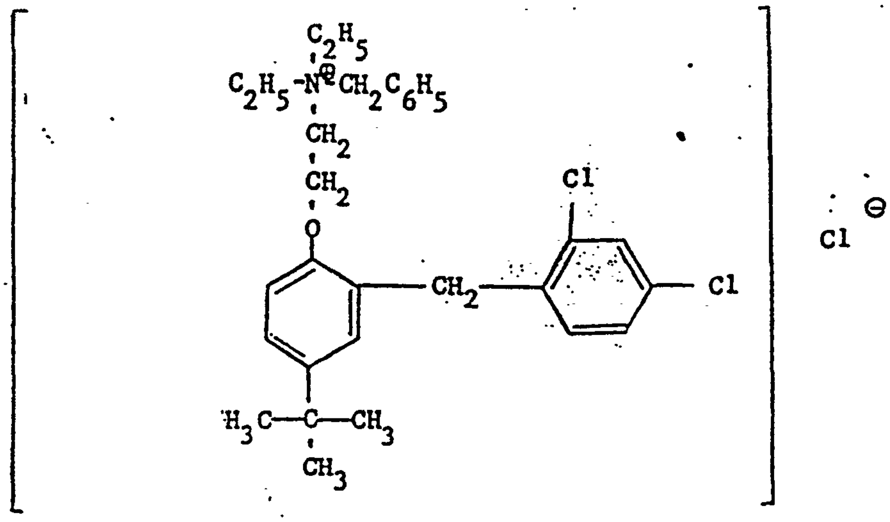

(exemple 29; n° de code: B 674).

On fait réagir dans un réacteur 1 mole (408,25 g) de β-[2-(2,4-dichlorobenzyl)-4-tertiobutylphénoxy]-N,N-diéthyl-éthyl amine avec 1,25 mole de chlorure de benzyle pendant 17 heures à 90-100°C sous agitation. Après refroidissement jusqu'à la température ambiante (15-20°C) on lave le milieu réactionnel avec le diéthyléther pour obtenir un précipité qu'on lave à l'hexane puis à l'acétone à froid (15-20°C). Par recristallisation on obtient le B 674 avec un rendement de 85,8 %.

$F_{inst}$ = 120-125°C. Préparation III

Obtention du chlorhydrate de β-[2-(2,4-dichlorobenzyl)-4-(-1,1-diméthylpropyl)-phénoxy]-N,N-diéthyl-éthylamine.

(exemple 14; n° de code; B 785)

Dans une solution de 1 mole (422,31 g) de β-[2-(2,4-dichlorobenzyl)-4-(1,1-diméthylpropyl)-phénoxy]-N,N-diethyl-éthylamine (exemple 6; n° de code; B 748) dans 800 ml de diéthyléther anhydre on fait barboter un courant de HCl gaz. On évapore sous vide jusqu'à siccité reprend le résidu d'évaporation avec du diéthyléther anhydre. Il se forme un précipité qu'on lave avec du diéthyléther anhydre. On obtient le B 785 avec un rendement de 83,3

$F_{inst}$ = 145-150°C. Préparation IV

Obtention du iodure de N,N-diéthyl-N-{2-[2-(2,4-dichlorobenzyl-)-4-(1,1-diméthyleropyl)-phénoxy]-éthyl}-N-méthylammonium.

(exemple 21; n° de code: B 794)

On fait réagir pendant 2 heures à 45°C sous agitation un mélange de 1 mole (422,31 g) de β-[2-(2,4-dichlorobenzyl)-4-(1,1-diméthylpropyl)-phénoxy]-N,N-diéthyl-éthylamine, de 1,25 mole de $ICH_3$ et 1,2 l d'acétone anhydre. Après refroidissement on verse le milieu réactionnel sur $CH_3CO_2C_2H_5$ froid (15°C). On obtient un précipité. Par recristallisation dans le mélange $CH_3CO_2C_2H_5$ - $C_2H_5OH$ (98: 2) v/v on obtient le B 794 avec un rendement de 62,4 %.

$F_{inst}$ = 138-140°C. Préparation V

Obtention du bromure de N,N-diéthyl-N-{2-[2-(2,4-dichlorobenzyl)-4-(1,1-diméthylpropyl)-phénoxy]-éthyl}-N-butylammonium.

(exemple 23; n° de code: B 798).

On fait réagir 1 mole (422,31 g) de β-[2-(2,4-dichloro-benzyl)-4-(1,1-diméthylpropyl)-phénoxy]-N,N-diéthyléthylamine avec 1,25 mole de 1-bromobutane pendant 15 heures à 100°C sous agitation. Après refroidissement on lave le milieu réactionnel avec de l'hexane puis avec du diéthyléther pour obtenir un précipité. Par lavage du précipité à 30°C avec $CH_3CO_2C_2H$, on obtient le B 798 avec un rendement de 20,4 %.

$F_{inst}$ = 104-108°C.

**Revendications** pour les Etats contractants: BE, CH, DE, FR, GB, IT, LI,

LU, NL, SE

1. Dérivé de β-[-2-(halogénobenzyl)-phénoxy]-éthylamine caractérisé en ce qu'il est choisi parmi l'ensemble constitué par

(i) les N,N-dialkyl-β-[ 2-(polychlorobenzyl)-4-alkylphénoxyl]-éthylamines répondant à la formule générale

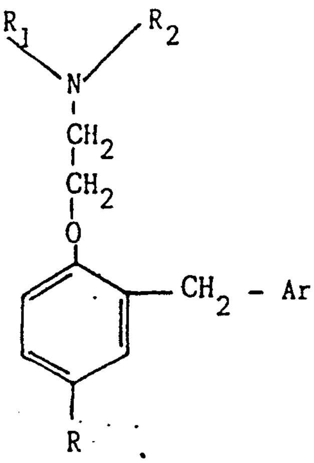

(I)

dans laquelle

R représente un groupe alkyle en $C_1$-$C_8$ à chaîne hydrocarbonée linéaire ou ramifiée,

$R_1$ et $R_2$, identiques ou différents, représentent chacun un groupe alkyle en $C_1$-$C_4$, et

Ar représente un groupe polychlorophényle de formule

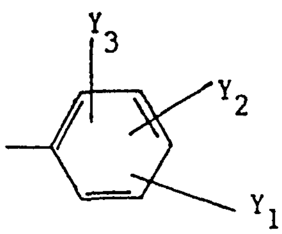

(II)

(où $Y_1$ est 4-Cl, $Y_2$ est H ou Cl, et $Y_3$ est H ou Cl, un au moins des $Y_2$ et $Y_3$ étant Cl); et

(ii) leurs sels d'addition.

2. Dérivé selon la revendication 1, caractérisé en ce que R est un groupe $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_3CH_3$, $(CH_2)_5CH_3$, $CH_2C(CH_3)_3$, $CH_2CH(CH_3)CH_2CH_3$, $C(CH_3)_2CH_2C(CH_3)_3$.

3. Dérivé selon la revendication 1, caractérisé en ce qu'il est choisi parmi l'ensemble constitué par les sels d'ammonium de formule

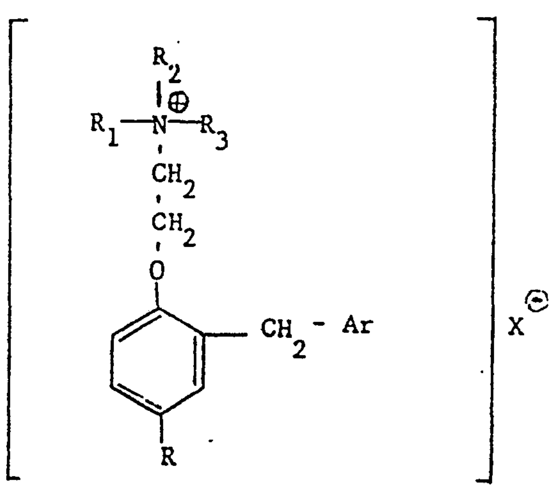

(I')

où Ar, R, $R_1$ et $R_2$ sont définis comme indiqué dans la revendication 1, $R_3$ est un groupe alkyle en $C_1$-$C_{10}$ ou un groupe benzylé, et, X est F, Cl, Br ou I.

4. Dérivé selon la revendication 1, caractérisé en ce qu'il est choisi parmi l'ensemble constitué par (i) les composés de formule

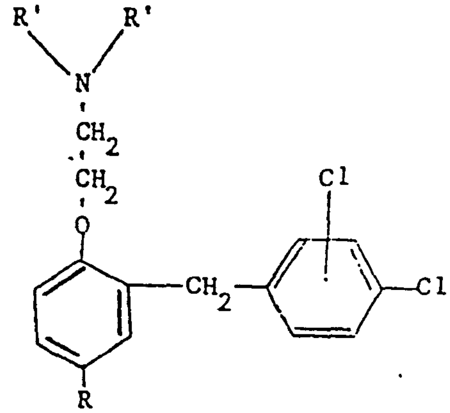

(III)

où R' est $CH_3$ ou $C_2H_5$ et R est un groupe alkyle en $C_1$-$C_8$; et, (ii) leurs sels d'addition d'acide.

5. Dérivé selon la revendication, caractérisé en ce qu'il est choisi parmi l'ensemble constitué par les sels d'ammonium de formule

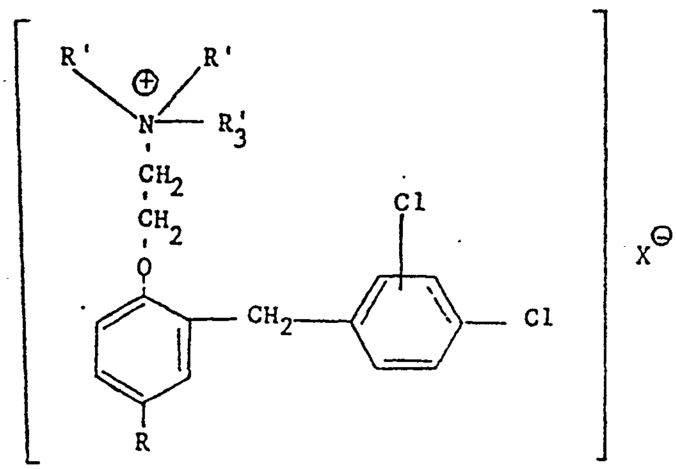

(III')

où R' est $CH_2$, $C_2H_5$, R est un groupe alkyle en $C_1$-$C_8$, $R'_3$ est $CH_3$, $CH_2CH_3$ ou $CH_2C_6H_5$, et X est F, Cl, Br ou I.

6. β-[2-(2,4-Dichlorobenzyl)-4-tertiobutylphénoxy]-N,N-diéthyl-éthylamine et ses sels d'addition.

7. β-[2-(2,4-Dichlorobenzyl)-4-(1,1-diméthylpropyl)-phénoxy]-N N-diéthyl-éthylamine et ses sels d'addition.

8. Chlorure de N,N-diéthyl-N-{2-[2-(2,4-dichlorobenzyl)-4-tertiobutylphénoxy]-éthyl}-N-benzylammonium.

9. 2-[2,4-Dichlorobenzyl)-4-(1,1,3,3-tétraméthyl-butyl)-phénoxy]-N,N-diéthyl-éthylamine et ses sels d'addition.

10. Chlorure de N,N-diéthyl-N-{2-[2-(2,4-dichlorobenzyl)-4-(1,1,3,3-tétraméthylbutyl)-phénoxy]-éthyl}-N-benzylammonium.

11 Composition thérapeutique caractérisée en ce qu'elle renferme, en association avec un excipient physiologiquement, au moins un composé de formule I ou l'un de ses sels d'addition selon l'une quelconque des revendications 1 à 10.

12. Procédé de préparation d'un composé de formule 1 ou l'un de ses sels d'addition selon la revendication 1, caractérisé en ce que l'on fait réagir un 2-(halogénobenzyl)-4-alkylphénol de formule

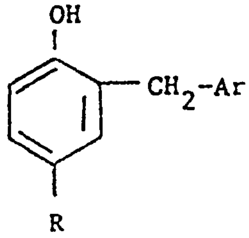

(IV)

(où R et Ar sont définis comme indiqué ci-dessus) avec un halogénure de β-N,N-dialkylaminoéthyle de formule

$Hal\text{-}CH_2\text{-}CH_2\text{-}NR_1R_2$ (V)

(où Hal est Cl ou Br, et, $R_1$ et $R_2$ sont définis comme indiqués ci-dessus) dans un solvant inerte, pendant au moins 5 heures à une température, inférieure ou égale à la température de reflux du milieu réactionnel si nécessaire, les sels d'addition d'acide étant ensuite obtenus par réaction de la base libre avec un acide minéral ou organique, et les sels d'ammonium étant obtenus par réaction de la base libre avec un halogénure $R_3X$ (où X est F, Cl, Br ou I, et, $R_3$ est un groupe alkyle en $C_1$-$C_{10}$ ou un groupe benzyle).

**Revendications** pour l'Etat contractant: AT

1. Procédé de préparation d'un nouveau dérivé de β-[-2(halogénobenzyl)-phénoxy]-éthylamine répondant à la formule générale

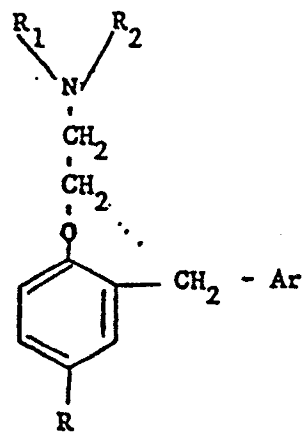

(I)

où

R représente un groupe alkyle en $C_1$-$C_8$ à chaîne-hydrocarbonée linéaire ou ramifiée,

$R_1$ et $R_2$, indentiques ou différents, représentent chacun un groupe alkyle en $C_1$-$C_4$, et Ar réprésente un groupe polychlorophénylé de formule

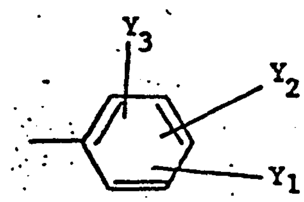

(II)

(où $Y_1$ est 4-Cl, $Y_2$ est H ou Cl, et $Y_3$ est H ou Cl, un au moins des $Y_2$ et $Y_3$ étant Cl);

et de ses sels d'addition;

ledit procédé étant caractérisé en ce que l'on fait réagir un 2-(polychlorobenzyl)-4,-alkylphénol de formule

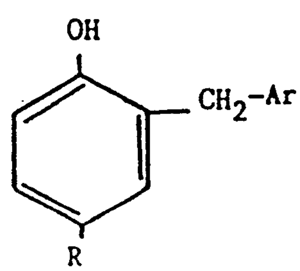

(IV)

(où R et Ar sont définis comme indiqué ci-dessus) avec un halogénure de formule

$Hal-CH_2-CH_2-NR_1R_2$ (V)

(où Hal est Cl ou Br, et, $R_1$ et $R_2$ sont définis comme indiqué ci-déssus) dans un solvant inerte, pendant au moins 5 h à une température inférieure ou égale à la température de reflux du milieu réactionnel, puis, si nécessaire, on prépare un sel d'addition d'acide, par réaction de la base libre ainsi obtenue, avec un acide minérale ou organique, et, on prépare un sel d'ammonium, par réaction de ladite base libre avec halogénure $R_3X$ (où X est F, Cl, Br ou I, et, $R_3$ est un groupe alkyle en $C_1$-$C_{10}$ ou un groupe benzyle).

2. Procédé selon la revendication 1, caractérisé en ce que l'on fait réagir au moins 1,1 mole d'halogénure de formule V pour 1 mole de 2-(polychlorobenzyl)-4-alkylphénol de formule IV.

3. Procédé selon la revendication 1, caractérisé en ce que le solvant inerte est choisi parmi l'ensemble constitué par le tétrahydrofuranne et le diméthylformamide.

4. Procédé selon la revendication 1, caractérisé, en ce que R est $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_3CH_3$, $(CH_2)_5CH_3$, $CH_2C(CH_3)_3$, $CH_2CH(CH_3)CH_2CH_3$ ou $C(CH_3)_2CH_2C(CH_3)_3$.

5. Procédé selon la revendication 1, caractérisé en ce que $R_3$ est $CH_3$, $CH_2CH_3$ ou $CH_2C_6H_5$.

6. Procédé selon la revendication 1, caractérisé en ce que Ar est 2,4-dichlorophényle.

**Patentansprüche** für die Vertragsstaaten: BE, CH, DE, FR, GB, IT, LI,

LU, NL, SE

1. Derivate vom β-[2-(Halogenbenzyl)-phenoxy]-ethylamin, dadurch gekennzeichnet, daß sie ausgewählt sind aus der Gruppe der

(i) N,N-Dialkyl-β-[-2-(polychlorbenzyl)-4-alkylphenoxy]-ethylamine entsprechend der allgemeinen Formel

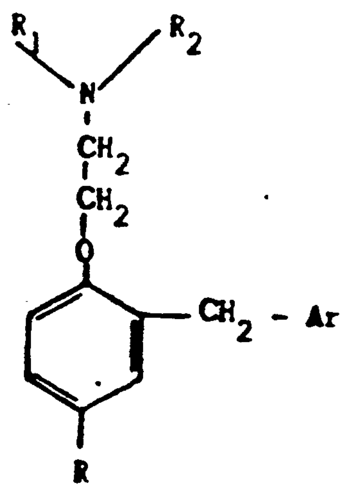

(I)

in welcher R eine gerad- oder verzweigtkettige $C_1$-$C_8$-Alkylgruppe bedeutet $R_1$ und $R_2$ die gleiche oder eine verschiedene $C_1$-$C_4$-Alkylgruppe darstellen und Ar einer Polychlorphenylgruppe der Formel

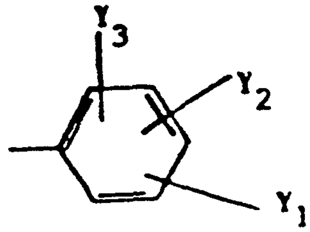

(II)

entspricht, worin $Y_1$ ein 4-Cl, $Y_2$ H oder Cl und Y3 H oder Cl ist, jedoch mindestens ein $Y_2$ und $Y_3$ Cl darstellt, und der

(ii) Additionssalze derselben.

2. Derivate nach Anspruch 1, dadurch gekennzeichnet, daß R bedeutet: $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_3CH_3$, $(CH_2)_5CH_3$, $CH_2C(CH_3)_3$, $CH_2CH(CH_3)CH_2CH_3$, $C(CH_3)_2CH_2C(CH_3)_3$.

3. Derivate nach Anspruch 1, dadurch gekennzeichnet, daß sie ausgewählt sind aus der Reihe der Ammonium

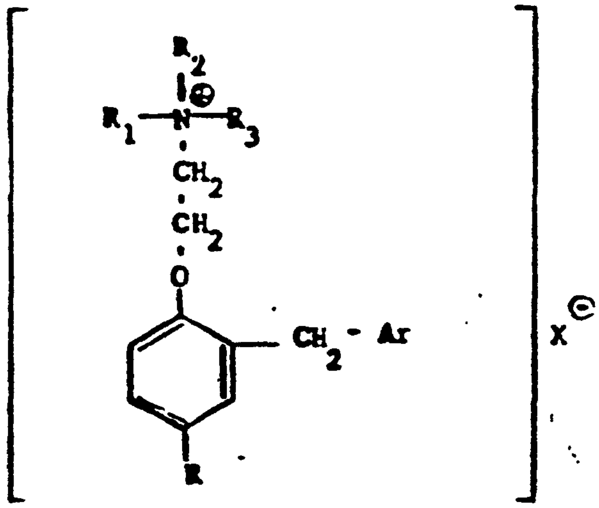

(I')

worin Ar, R, $R_1$ und $R_2$ der im Anspruch 1 angegebenen Definition entsprechen, $R_3$ eine $C_1$-$C_{10}$-Alkylgruppe oder eine Benzylgruppe darstellt und X entweder F, Cl, Br oder I ist.

4. Derivate nach Anspruch 1, dadurch gekennzeichnet, daß sie ausgewählt sind aus der Reihe der

(i) Verbindungen der Formel

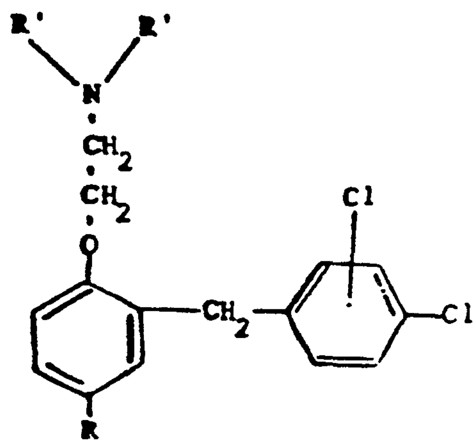

(III)

worin R' $CH_3$ oder $C_2H_5$ ist und R eine $C_1$-$C_8$-Alkylgruppe bedeutet und der

(ii) Säureadditionssalze derselben.

5. Derivate nach Anspruch 1, dadurch gekennzeichnet, daß sie ausgevählt sind aus den Ammoniumsalzen der Formel

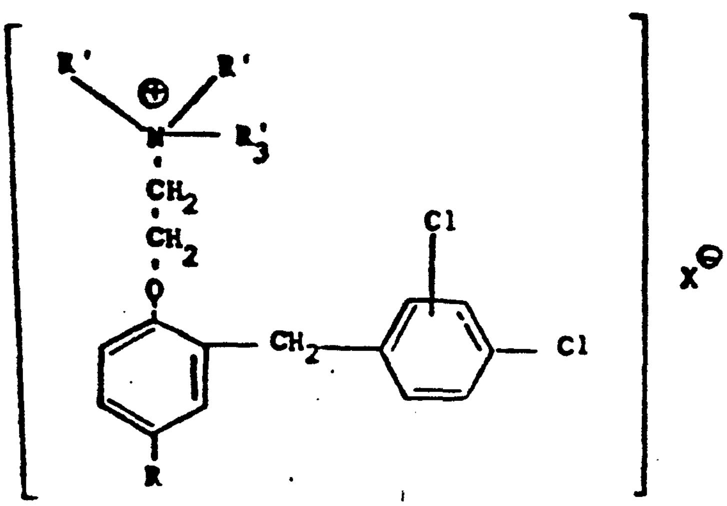

(III')

worin R' $CH_3$ oder $C_2H_5$ ist, R eine $C_1$-$C_8$-Alkyl-gruppe darstellt, $R_3$' $CH_3$, $CH_2CH_3$ oder $CH_2C_6H_5$ bedeutet und X entweder F, Cl, Br oder I ist.

6. β-[2-(2,4-Dichlorbenzyl)-4-tert. butylphenoxy]-N,N-diethyl-ethylamin und seine Additionssalze.

7. β-[2-(2,4-Dichlorbenzyl)-4-(1,1-dimethylpropyl)-phenoxy] -N,N-diethyl-ethylamin und seine Additionssalze.

8. N,N-Diethyl-N-{2-[2-(2,4-dichlorbenzyl)-4-tert. butylphenoxy]-ethyl}-N-benzylammoniumchlorid.

9. β-[2-(2,4-Dichlorbenzyl)-4-(1,1,3,3-tetramethyl-butyl)-phenoxy]-N,N-diethyl-ethylamin und seine Additionssalze.

10. N,N-Diethyl-N-{-2-[2-2,4-dichlorbenzyl)-4-(1,1,3,3-tetramethylbutyl)-phenoxy]-ethyl}-N-benzylammonium-chlorid.

11. Therapeutische Zusammensetzung, dadurch gekennzeichnet, daß sie zusammen mit einer physiologischen Grundmasse mindestens eine Verbindung entsprechend der Formel I oder eines der Additionssalze nach irgendeinem der Ansprüche 1 bis 10 enthält.

12. Verfahren zur Herstellung einer Verbindung der Formel oder eines Additionssalzes davon entsprechend Anspruch 1, dadurch gekennzeichnet, daß man ein 2-(Halogenbenzyl)-4-alkylphenol der Formel

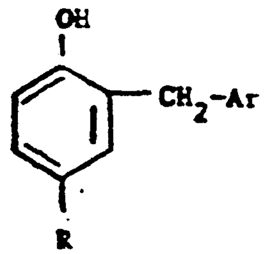

(IV)

(worin R und Ar der obigen Definition entsprechen) mit einem β-N,N-Dialkylaminoethyl-halogenid der Formel

Hal-$CH_2$-$CH_2$-$NR_1R_2$ (V)

worin Hal Cl oder Br ist und $R_1$ sowie $R_2$ die oben angeführte Bedeutung haben, in einem inerten Lösungsmittel während mindestens 5 Stunden bei einer Temperatur, die niedriger oder gleich der Rückflußtemperatur des Reaktionsmilieus ist, reagieren läßt und ggfls. die Säure-Additionssalze durch Umsatz der freien Base mit einer mineralischen oder organischen Säure sowie die Ammoniumsalze durch Umsatz der freien Base mit einem Halogenid $R_3X$, vorin X F, Cl, Br oder I ist und $R_3$ einer C1-C10-Alkylgruppe oder einer Benzylgruppe entspricht, gewinnt.

**Patentansprüche** für den Vertragsstaat: AT

1. Verfahren zur Herstellung von neuen Derivaten vom β-[2-(Halogenbenzyl) -phenoxy]-ethylamin entsprechend der allgemeinen Formel

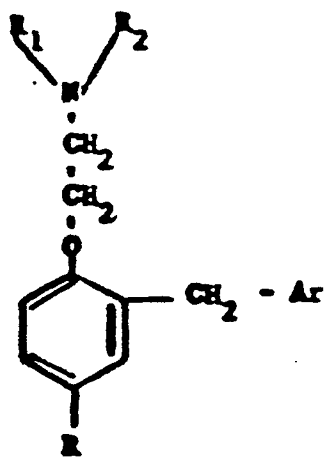

(I)

in der R eine lineare oder verzweigtkettige $C_1$-$C_8$-Alkylgruppe ist, $R_1$ und $R_2$ jeweils denselben oder einen anderen $C_1$-$C_4$-Alkylrest darstellen und Ar eine Polychlorphenylgruppe der Formel

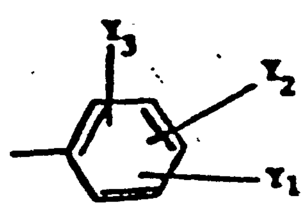

(II)

bedeutet, worin $Y_1$ 4-Cl, $Y_2$ H oder Cl und $Y_3$ H oder Cl sind, jedoch mindestens einer der Reste $Y_2$ und $Y_3$ Cl ist, dadurch gekennzeichnet, daß man ein 2-(Polychlorbenzyl)-4-alkylphenol der Formel

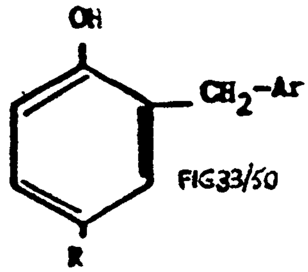

(IV)

FIG33/50

worin R und Ar der obigen Definition entsprechen, mit einem Halogenid der Formel

$Hal-CH_2-CH_2NR_1R_2$ (V)

in der Hal Cl oder Br ist und $R_1$ sowie $R_2$ die oben angeführte Bedeutung haben, in einem inerten Lösungsmittel während mindestens 5 Stunden bei einer Temperatur, die niedriger oder gleich der Rückflußtemperatur des Reaktionsmilieus ist, reagieren läßt und ggfls. die Säureadditionssalze durch Umsatz der so erhaltenen freien Basen mit einer mineralischen oder organischen Säure sowie die Ammoniumsalze durch Umsatz der genannten freien Basen mit einem Halogenid $R_3X$, worin X entweder F, Cl, Br oder I ist und $R_3$ eine $C_1$-$C_{10}$-Alkylgruppe oder eine Benzylgruppe darstellt, gewinnt.

2. Verfahren nach Anspruch 1, dadurch gekennzeichnet, daß man mindestens 1,1 Mol des Halogenids der Formel V je Mol des 2-(Polychlorbenzyl)-4-alkylphenols der Formel IV miteinander reagieren läßt.

3. Verfahren nach Anspruch 1, dadurch gekennzeichnet, daß das inerte Lösungsmittel ausgewählt wird aus der aus Tetrahydrofuran und Dimethylformamid gebildeten Gruppe.

4. Verfahren nach Anspruch 1, dadurch gekennzeichnet, daß R $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_3CH_3$, $(CH_2)_5CH_3$, $CH_2C(CH_3)_3$, $CH_2CH(CH_3)CH_2CH_3$ oder $C(CH_3)_2CH_2C(CH_3)_3$ ist.

5. Verfahren nach Anspruch 1, dadurch gekennzeichnet, daß $R_3$ $CH_3$, $CH_2CH_3$ oder $CH_2C_6H_5$ ist.

6. Verfahren nach Anspruch 1, dadurch gekennzeichnet, daß Ar ein 2,4-Dichlorphenylrest ist.

**Claims** for the contracting states: BE, CH, DE, FR, GB, IT, LI, LU, NL,

SE.

1. A β-[2-(halogenobenzyl)-phenoxy]-ethylamine derivative characterized in that it is selected from the group consisting of

(i) N,N-dialkyl-β-[2-(polychlorobenzyl)-4-alkylphenoxy]-ethylamines of the general formula

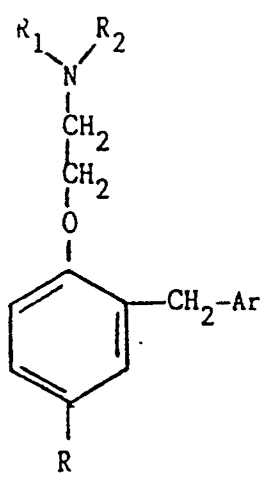

(I)

wherein

R represents a $C_1$-$C_8$-alkyl group having a linear or branched hydrocarbon chain,

$R_1$ and $R_2$, which may be identical or different, represent each a $C_1$-$C_4$-alkyl group, and

Ar represent a polychlorophenyl group of the formula

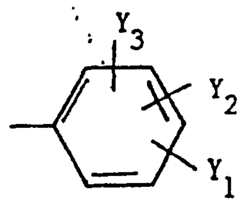

(II)

(in which $Y_1$ is 4-Cl, $Y_2$ is H or Cl, and $Y_3$ is H or Cl, at least one of the $Y_2$ and $Y_3$ being Cl); and
(ii) their addition salts.

2. A compound according to claim 1 in which R is $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_5CH_3$, $(CH_2)_3CH_3$, $CH_2C(CH_3)_3$, $CH_2CH(CH_3)CH_2CH_3$ or $C(CH_3)_2CH_2C(CH_3)_3$.

3. A compound according to claim 1 which is selected from the group consisting of ammonium salts of the formula

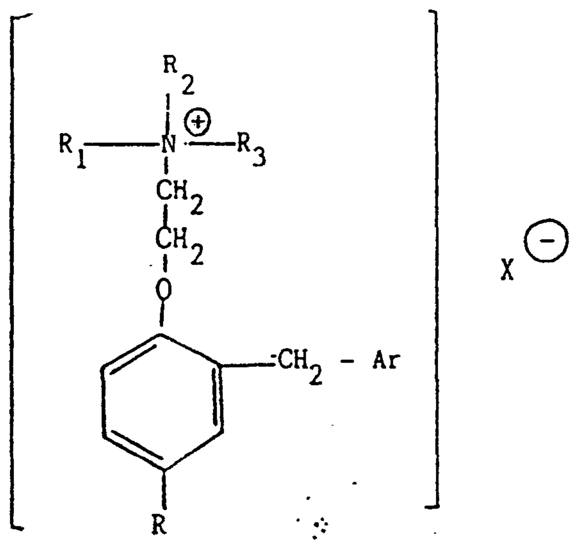

(I')

wherein Ar, R, $R_1$ and $R_2$ are defined as indicated in claim 1, $R_3$ is a $C_1$-$C_{10}$-alkyl or benzyl group, and, X is F, Cl, Br or I.

4. A compound according to claim I, which is selected from the group consisting of
(i) compounds of the formula

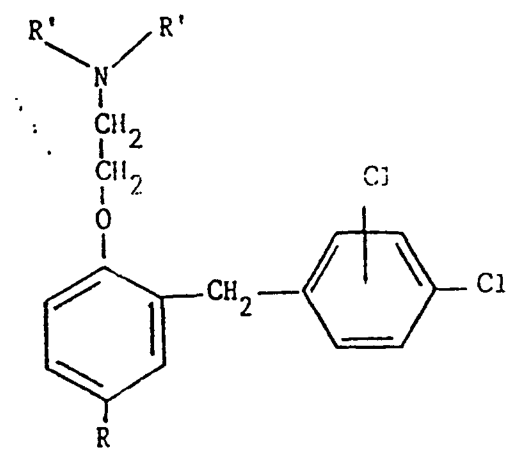

(III)

wherein R' is $CH_3$ or $C_2H_5$ and R is a $C_1$-$C_8$-alkyl group; and
(ii) acid addition salts thereof.

5. A compound according to claim 1 which is selected from the group consisting of ammonium salts of the formula

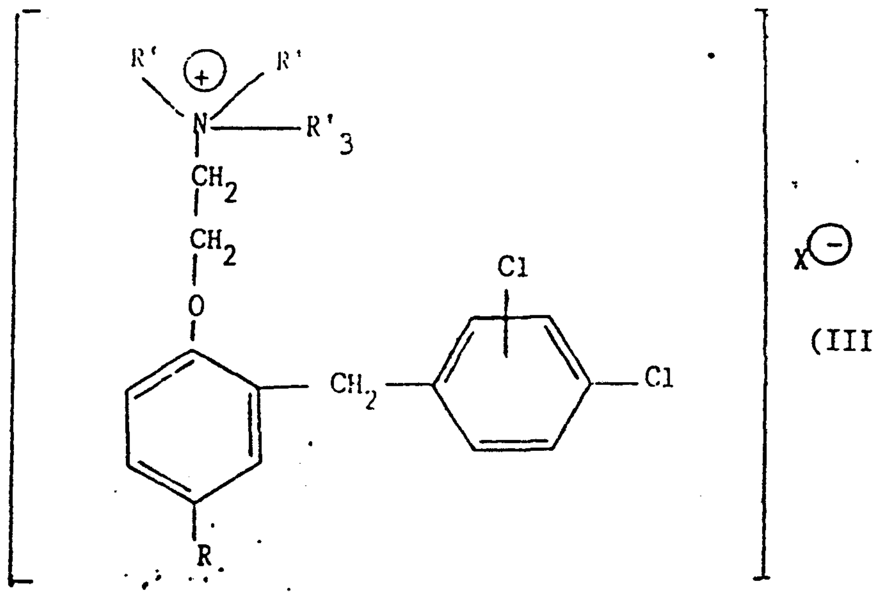

(III')

wherein R' is $CH_3$ or $C_2H_5$, R is a $C_1$-$C_8$-alkyl group, $R'_3$ is $CH_3$, $CH_2CH_3$ or $CH_2$ $C_6H_5$, and X is F, Cl, Br or I.

6. β-[-2-(2,4-Dichlorobenzyl-4-t-butylphenoxy]-N N-diethylethylamine and its addition salts.

7. β-[-2-(2-(2,4-Dichlorobenzyl)-4-(1,1-dimethylpropyl)-phenoxy]-N,N-diethylethylamine and its addition salts.

8. N,N-Diethyl-N-{2-[2-(2,4-dichlorobenzyl)-4-t-butylphenoxy]-ethyl}-N-benzylammonium chloride.

9. β-[2-(2,4-Dichlorobenzyl)-4-(1,1,3,3-tetramethylbutyl)-phenoxy]-N,N-diethylethylamine and its addition salts.

10. N,N-Diethyl-N-{2-[2-(2,4-dichlorobenzyl)-4-(1,1,3,3-tetramethyl-butyl)-phenoxy]-ethyl}-N-benzylammonium chloride.

11. A therapeutical composition comprising, in association with a physiologically acceptable excipient, a pharmaceutically effective amount of a compound according to claim 1.

12. A method for preparing a compound of the formula 1 or one of its addition salts according to claim 1, said method comprising reacting a 2-(polyhalogenobenzyl)-4-alkylphenol of the formula

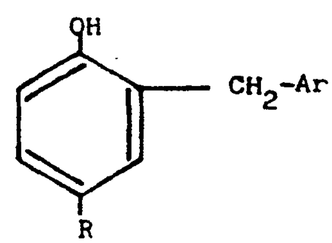

(IV)

(wherein R and Ar are defined as indicated above) with a β -N,N-dialkylaminoethyl halide of the formula

Hal-$CH_2$-$CH_2$-$NR_1R_2$ (V)

(wherein Hal is Cl or Br, and, $R_1$ and $R_2$ are defined as indicated above), in an inert solvent for at least 5 hours at a temperature lower than or equal to the refluxing temperature of the reaction mixture; and,

if needed, the acid addition salt is obtained by reacting the free base thus obtained with an organic or inorganic acid, and the ammonium salt is obtained by reacting said free base with an halide $R_3X$ (wherein X is F, Cl, Br or I, and, $R_3$ is $C_1$-$C_{10}$-alkyl or benzyl).

**Claims** for the contracting state: AT

1. A method for preparing a new β-[2-(halogenobenzyl)-phenoxy]-ethylamine derivative of the general formula

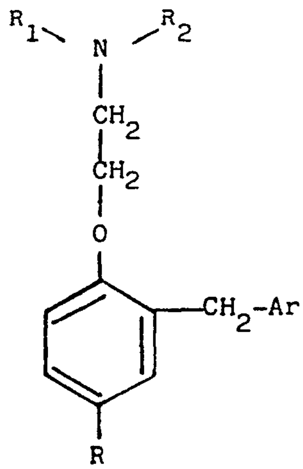

(I)

wherein
$R_1$ represents a $C_1$-$C_8$-alkyl group having a linear or branched hydrocarbon chain,
$R_1$ et $R_2$, which may be identical or different, represent each a $C_1$-$C_4$-alkyl group, and
Ar represents a polychlorophenyl group of the formula

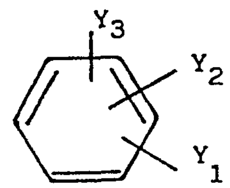

(II)

(in which $Y_1$ is 4-Cl, $Y_2$ is H or Cl, and $Y_3$ is H or Cl, at least one of the $Y_2$ and $Y_3$ being Cl); and addition salts thereof, said method comprising reacting a 2-(polyhalogenobenzyl)-4-alkylphenol of the formula

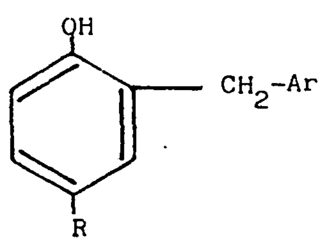

(IV)

(wherein R and Ar are defined as indicated above) with a β-N,N-dialkylaminoethyl halide of the formula
$Hal-CH_2-CH_2-NR_1R_2$ (V)
(wherein Hal is Cl or Br, and, $R_1$ and $R_2$ are defined as indicated above), in an inert solvant for at least 5 hours at a temperature lower than or equal to the refluxing temperature of the reaction mixture; and,

if needed, the acid addition salt is obtained by reacting the free base thus obtained with an organic or inorganic acid, and the ammonium salt is obtained by reacting said free base with an halide $R_3X$ (wherein X is F, Cl, Br or I, and, $R_3$ is $C_1$-$C_{10}$-alkyl or benzyl).

2. A method according to claim 1, characterized in that 1 mol of 2-(polychlorobenzyl)-4-alkylphenol of the formula IV is reacted with at least 1.1 mol of halide of the formula V.

3. A method according to claim 1, characterized in that the inert solvent is selected from the group consisting of tetrahydrofuran and dimethylformamid.

4. A method according to claim 1, characterized in that R is $CH_3$, $CH_2CH_3$, $CH(CH_3)_2$, $C(CH_3)_3$, $CH(CH_3)CH_2CH_3$, $C(CH_3)_2CH_2CH_3$, $(CH_2)_3CH_3$, $(CH_2)_5CH_3$, $CH_2C(CH_3)_3$, $CH_2CH(CH_3)CH_2CH_3$ or $C(CH_3)_2CH_2C(CH_3)_3$.

5. A method according to claim 1, characterized in that R is $CH_3$, $CH_2CH_3$ or $CH_2C_6H_5$.

6. A method according to claim 1, characterized in that Ar is 2,4-dichlorophenyl.